# EUROPEAN PATENT APPLICATION

(11) **EP 3 718 998 A1**
(43) Date of publication of application: **07.10.2020**
(21) Application number: 18882596.2
(22) Date of filing: 01.11.2018
(51) Int. Cl.: C07C 67/303, C07C 69/75, B01J 23/46, C08K 5/00, C08K 5/101, C08L 101/00

(54) **METHOD FOR HYDROGENATION OF PHTHALATE COMPOUND**

(30) Priority: 29.11.2017 KR 20170161952
(71) Applicant: Hanwha Solutions Corporation, Jung-gu Seoul 04541 (KR)
(72) Inventor: JUNG, Jae Heum, Busan 48050 (KR); JUNG, Ki Taeg, Daejeon 34199 (KR); KIM, Hyo Suk, Daejeon 34023 (KR); PARK, Seong Min, Seoul 02105 (KR); LEE, Kyoung Il, Daejeon 34207 (KR); LEE, Hye Won, Daejeon 305-720 (KR)
(74) Representative: Gevers Patents
(86) International application number: PCT/KR2018/013197
(87) International publication number: WO 2019/107773

(57) **Abstract**

The invention relates to a method for hydrogenation of a phthalate compound. According to the method, stereoselectivity of hydrogenation increases and the content of cis isomers in the hydrogen product increases, and thus the quality of the product as a plasticizer may be improved.

## Description

### [Technical Field]

### Cross-reference to Related Application

This application claims the benefit of Korean Patent Application No. 10-2017-0161952 filed on November 29, 2017 with the Korean Intellectual Property Office, the disclosures of which are herein incorporated by reference in their entirety.

The invention relates to a method for the hydrogenation of a phthalate compound. Specifically, it relates to a method for the hydrogenation of a phthalate compound that may increase stereoselectivity of the reaction to increase the content of cis isomers in the hydrogen product.

### [Background Art]

A phthalate based compound is material widely used as a plasticizer of plastic, particularly polyvinylchloride(PVC). For example, it is used for various applications such as electrical and electronic products, medicine, paint, lubricant, binder, surfactant, adhesive, tile, food containers, packaging material, and the like.

However, several phthalate compounds are known to cause environmental pollution and endocrine disruption of human, and the regulation of utilization is being strengthened around advanced countries such as Europe, the US, and the like. Particularly, among the phthalate-based plasticizers, some products such as di(2-ethylhexyl) phthalate (DEHP), butyl benzyl phthalate (BBP), or di-n-butyl phthalate (DBP) are suspected as endocrine disruptors that disturb or confuse the hormone action of human, and thus, there is a move for the regulation of the same.

Thus, efforts to develop environmentally friendly plasticizers that exhibit equivalent performance to the conventional plasticizers but are free from environmental hormone problem are being made, and one of them is a method of using a compound in which a benzene ring included in a phthalate compound is hydrogenated.

As the hydrogenation of an aromatic compound such as a benzene ring, a method of using a catalyst including a transition metal such as ruthenium as an active ingredient on a carrier, is known. For example, Korean Registered Patent No. 1556340 suggests a hydrogenation method of reacting a phthalate compound with hydrogen in the presence of a hydrogen catalyst and alcohol, and discloses that catalyst performance and life are improved according to this method.

The product prepared by the hydrogenation is obtained as a mixture of cis and trans isomers. Herein, as the content of cis isomers is higher, excellent plasticization efficiency for PVC resin, rapid absorption speed, and high product transparency after solidification are exhibited, and leaching on the product surface is less generated even after use for a long time, thus exhibiting excellent properties as a plasticizer.

Thus, in order to obtain a plasticizer having excellent quality, a hydrogenation method of a phthalate compound with improved stereoselectivity such that the content of cis isomers is high is required.

### [Prior Art Technical Document]

Patent Document 1: Korean Registered Patent No. 1556340, "A Method of Hydrogenation of Phthalate Compound"

### [Disclosure]

### [Technical Problem]

In order to solve the above problem, it is an object of the invention to provide a novel method for the hydrogenation of a phthalate compound that may increase the content of cis isomers in the hydrogen product.

### [Technical Solution]

In order to achieve the object, there is provided a method for the hydrogenation of a phthalate compound including the step of reacting a phthalate compound with hydrogen in the presence of a hydrogenation catalyst in a reactor,
wherein a temperature deviation in the reactor is 0 to 30 °C during the reaction, and
the content of cis isomers in the hydrogenation product separated after the reaction is 70 % or more.

Herein, during the reaction, a temperature deviation per unit length (m) of the reactor may be 10 °C or less.

During the reaction, the reaction amount per unit volume (m³) of the reactor during the reaction may be 100 kmol/h or less.

The reactor may include a cooling member in which coolant is circulated, installed outside.

The mass flux per unit area (m²) of the phthalate compound introduced into the reactor may be 10,000 to 100,000 kg^{∗}h^{-1∗}m⁻².

The amount of hydrogen introduced into the reactor may be 3 to 300 mol per 1 mol of the phthalate compound.

The phthalate compound may be one or more selected from the group consisting of phthalate, terephthalate, isophthalate, and carboxylic acid compounds thereof.

The gas phase raw material may be fed from the upper part or lower part of the reactor, and the liquid raw material may be fed from the upper part of the reactor.

The hydrogenation catalyst may be one or more selected from the group consisting of ruthenium (Ru), rhodium (Rh), palladium (Pd), and platinum (Pt).

The amount of the hydrogenation catalyst may be 3 wt% or less, based on 100 wt% of a carrier.

There is also provided a hydrogenated phthalate or terephthalate compound, prepared by the above method.

The hydrogenated phthalate or terephthalate compound may be used as a plasticizer.

There is also provided a resin composition including the plasticizer, and a resin selected from ethylene vinyl acetate, polyethylene, polypropylene, polyvinyl chloride, polystyrene, polyurethane, polybutadiene, silicon, thermoplastic elastomer, or copolymers thereof.

### [Effect of the Invention]

According to the method of the invention, stereoselectivity of hydrogenation increases and the content of cis isomers in the hydrogen product increases, and thus the quality of the product as a plasticizer may be improved.

### [Brief Description of Drawings]

FIG. 1 briefly shows the hydrogenation apparatus used for the hydrogenation method of the invention.
FIG. 2 shows the results measuring the contents of cis isomers in the hydrogen products for examples and comparative examples.

### [Best Mode]

Although various modifications can be made to the present invention and the present invention may have various forms, specific examples will be illustrated and explained in detail below. However, it should be understood that these are not intended to limit the present invention to a specific disclosure, and that the present invention includes all modifications, equivalents, or replacements thereof without departing from the spirit and technical scope of the invention.

As used herein, terms including an ordinal such as "first", "second" and the like are used to explain various constructional elements, but the constructional elements are not limited by the terms. The terms are used only to distinguish one constructional element from other constructional elements. For example, a first constructional element may be called a second constructional element, and similarly, a second constructional element may be called a first constructional element.

A singular expression includes a plural expression thereof, unless it is expressly stated or obvious from the context that such is not intended. As used herein, the terms "comprise", "have", etc. are intended to designate the existence of practiced characteristics, numbers, steps, constructional elements, or combinations thereof, and they are not intended to preclude the possibility of existence or addition of one or more other characteristics, numbers, steps, constructional elements, or combinations thereof.

Hereinafter, a method for the hydrogenation of a phthalate compound of the invention will be explained in detail with reference to drawings.

According to one preferable embodiment of the invention, there is provided a method for the hydrogenation of a phthalate compound including the step of reacting a phthalate compound with hydrogen in the presence of a hydrogenation catalyst in a reactor,
wherein a temperature deviation in the reactor is 0 to 30 °C during the reaction, and
the content of cis isomers in the hydrogenation product separated after the reaction is 70 % or more.

By operating a reactor while controlling a temperature deviation of the reactor during the reaction, stereoselectivity of the hydrogenation of a phthalate compound is increased. Specifically, according to the method of the invention, the content of cis isomers is as high as 70 % or more in the hydrogenation product, and the product exhibits excellent plasticization efficiency for PVC resin, rapid absorption speed, and high product transparency after solidification, and generates less leaching on the product surface even after use for a long time, thus exhibiting excellent properties as a plasticizer. Thus, the invention may be utilized for the production of a plasticizer.

The hydrogenation subject is a phthalate compound, and by hydrogenation, hydrogen is added to the benzene ring of the phthalate compound, and the phthalate compound is converted into corresponding cyclohexane dicarboxylate.

The phthalate compound may be one or more selected from phthalate, terephthalate, isophthalate, and corresponding carboxylic acid.

First, the phthalate compound may be represented by the following Chemical Formula 1.

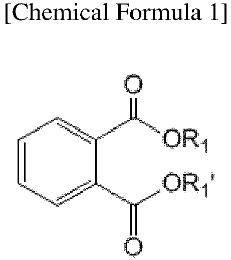

In Chemical Formula 1, R1 and R1' are identical to or different from each other, and are each independently hydrogen, or a C1 to 20, preferably a C4 to 20, more preferably a C5 to 20, even more preferably a C5 to 10, linear or branched alkyl group.

Specific examples of the phthalate compound may include dibutyl phthalate (DBP), dihexyl phthalate (DHP), dioctyl phthalate (DOP), di-n-octyl phthalate (DnOP), diisononyl phthalate, or diisodecyl phthalate (DIDP), but are not limited thereto. These compounds may be used alone or in combinations.

The terephthalate compound may be represented by the following Chemical Formula 2.

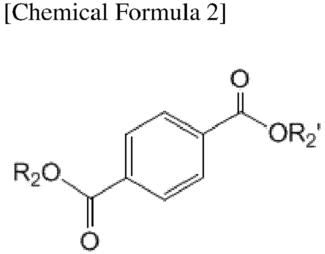

In Chemical Formula 2, R2 and R2' are identical to or different from each other, and are each independently hydrogen, or a C1 to 20, preferably a C4 to 20, more preferably a C5 to 20, even more preferably a C5 to 10 linear or branched alkyl group.

Specific examples of the terephthalate compound may include dibutyl terephthalate (DBTP), dioctyl terephthalate (DOTP), diisononyl terephthalate (DINTP), or diisodecyl terephthalate (DIDTP), but not are limited thereto. These compounds may be used alone or in combinations.

The isophthalate compound may be represented by the following Chemical Formula 3.

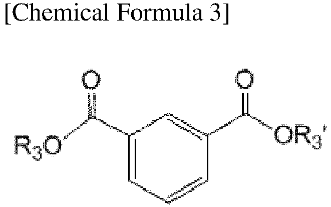

In Chemical Formula 3, R3 and R3' are identical to or different from each other, and are each independently hydrogen, or a C1 to 20, preferably a C4 to 20, more preferably a C5 to 20, even more preferably a C5 to 10 linear or branched alkyl group.

Specific examples of the isophthalate compound may include dibutyl isophthalate (DBIP), dioctyl isophthalate (DOIP), diisononyl isophthalate (DINIP), or diisodecyl isophthalate (DIDIP), but are not limited thereto. These compounds may be used alone or in combinations.

Preferably, as the phthalate compound, dioctyl terephthalate(DOTP) may be used.

The purity of the phthalate compound may be about 99 % or more, preferably about 99.5 % or more, more preferably about 98 % or more, but is not limited thereto, and commercially available phthalate compounds of any qualities and purities may be used.

The hydrogenation process of the phthalate compound may be conducted in a liquid phase or gas phase. According to one embodiment of the invention, the hydrogenation process may be progressed with the phthalate compound in a liquid state, and hydrogen in a gas state.

The mass flux of the phthalate compound introduced into the reactor per unit area (m²), namely, the mass flux per unit area (m²) of the reactor filled with a catalyst, may preferably be 10,000 to 100,000 kg^{∗}h^{-1∗}m⁻², and more preferably 17,000 to 50,000 kg^{∗}h^{-1∗}m⁻².

If the mass flux of the phthalate compound per unit area(m²) is less than 10,000 kg^{∗}h^{-1∗}m⁻², an input amount of raw material may not be sufficient, thus generating a decrease in productivity, and if it is greater than 100,000 kg^{∗}h^{-1∗}m⁻², the amount of liquid raw material introduced into the reactor at once may excessively increase, and the thickness of a raw material film on the catalyst surface may increase, and thus, it may be difficult for hydrogen to penetrate, and it may be difficult for a hydrogenation reaction to occur, and thus side reactions may increase and local heating may occur, thus increasing a temperature deviation of the reactor.

Meanwhile, in order to minimize side reactions and optimize the ratio between reaction materials to improve productivity, the amount of hydrogen introduced into the reactor may be 3 mol or more, or 4 mol or more, or 7 mol or more, and 300 mol or less, or 100 mol or less, or 50 mol or less, or 30 mol or less, based on 1 mol of the phthalate compound.

If the amount of hydrogen is less than 3 mol per 1 mol of the phthalate compound, reaction conversion may be lowered, and conversion of 95 % or more may not be obtained, and if it is greater than 300 mol, the residence time of liquid drops of the liquid raw material may be shortened by hydrogen, and thus, conversion may decrease or by-products may increase, or catalyst life may be rapidly shortened. In this regard, it is preferable that the amount of hydrogen is within the above range.

The temperature and pressure conditions of the gas phase raw material and liquid phase raw material introduced into the reactor are not specifically limited, but the gas phase raw material may be controlled to a pressure of about 100 to about 200 bar, preferably about 130 to about 160 bar, and a temperature of about 100 to about 200 °C, preferably about 130 to about 180 °C, and the liquid phase raw material may be controlled to a pressure of about 100 to about 200 bar, preferably about 130 to about 160 bar, and a temperature of about 100 to about 200 °C, preferably about 130 to about 180 °C.

The hydrogenation catalyst may include a transition metal as an active ingredient, and preferably, may include one or more selected from the group consisting of ruthenium (Ru), rhodium (Rh), palladium (Pd), and platinum (Pt).

Such a hydrogenation catalyst may be supported on a carrier, wherein any carriers known in the art may be used without limitations. Specifically, carriers such as zirconia (ZrO₂), titania (TiO₂), alumina (Al₂O₃), silica (SiO₂), and the like may be used.

In case the hydrogenation catalyst is supported on a carrier, the amount of the active ingredient of the hydrogenation catalyst may preferably be 3 wt% or less, 2 wt% or less, or 1 wt% or less, and 0.1 wt% or more, or 0.3 wt% or more, based on 100 wt% of the carrier. If the amount of the hydrogenation catalyst is greater than 3 wt% based on 100 wt% of the carrier, reactions may be rapidly progressed on the catalyst surface, and during the process, side reactions may also increase and by-products may rapidly increase, and if it is less than 0.1 wt%, due to an insufficient catalyst amount, the yield of a hydrogenation reaction may be lowered, and thus the above range is preferable.

The hydrogenation reaction conditions are not specifically limited, but for example, the reaction pressure may be 50 bar or more, or 100 bar or more, or 130 bar or more, and 220 bar or less, or 200 bar or less, or 180 bar or less. If the reaction pressure is less than 50 bar, a reaction may not easily occur, and thus an excessive amount of catalyst may be consumed, and residence time may excessively lengthen, thus increasing by-products, and if it is greater than 200 bar, excessive energy may be required during the operation of the process, and the manufacturing cost of equipment such as a reactor may significantly increase, and thus the above range is preferable.

Further, the reaction temperature may be 100 °C or more, or 120 °C or more, or 130 °C or more, and 300 °C or less, or 250 °C or less, or 200 °C or less. If the reaction temperature is less than 100 °C, reaction speed may be too slow, and thus the reaction may not smoothly occur, and if it is greater than 300 °C, by-products may rapidly increase. In addition, it may also have an influence on the catalyst life, and thus the above range is preferable.

By such a hydrogenation reaction, the aromatic ring of the phthalate compound is hydrogenated, and the phthalate compound is converted into a corresponding cyclohexane dicarboxylate compound.

The reaction is controlled such that a temperature deviation in the reactor may be maintained 0 to 30 °C, preferably 0 to 20 °C, and more preferably 0 to 10 °C during the hydrogen reaction, thereby minimizing the generation of trans isomers and improving selectivity to cis isomers. Herein, a temperature deviation means a difference between the maximum temperature and the minimum temperature in the reactor, and it may be measured by many temperature sensors installed according to the height of the reactor.

Alternatively, the reaction is controlled such that a temperature deviation per unit length (m) of the reactor is maintained 10 °C or less, or 5 °C or less, during the hydrogen reaction, thereby avoiding local heating and thus minimizing the generation of trans isomers and improving selectivity to cis isomers. The lower the temperature deviation per unit length of the reactor, the better, and thus the lower limit is not limited, but for example, the lower limit may be 3 °C or more, preferably 0 °C. Such a temperature deviation may be measured from many temperature sensors installed according to the height of the reactor.

And, according to one embodiment of the invention, the maximum reaction amount per the actual reaction volume(m³) of the reactor filled with catalysts is maintained at 100 kmol/h or less, or 50 kmol/h or less, preferably 30 kmol/h or less during the hydrogenation reaction, so as not to generate local heating in the reactor, thereby improving selectivity to cis isomers. Wherein, the reaction amount may be calculated through the concentration change value by respectively sampling the products according to the height of the reactor.

In order to control the reaction amount in the reactor, the mass flux or concentration of the reactant may be controlled as explained above, and for this purpose, an inert gas or inert liquid may be added together with the reactant, and the amount may be controlled.

A method for controlling a temperature deviation and reaction amount in the reactor is not specifically limited, and any methods known in the art may be used. For example, a method of installing a cooling member in which a coolant is circulated, inside or outside of the reactor, to diffuse reaction heat, may be used.

Herein, as the coolant, any coolants known in the art, such as cooling water, a methane based coolant, a mixed coolant of a fluorine-substituted or unsubstituted C1-5 lower alkane and ether, and the like, may be used without limitations. The kind of the cooling member is not specifically limited, but an indirect cooling method such as a heat exchanger, a cooling jacket, and the like, and a direct cooling method such as an inert gas, an inert liquid, and the like may be used.

In case the reaction is conducted while controlling a temperature deviation inside of the reactor, stereoselectivity of hydrogenation remarkably increases and the content of cis isomers in the hydrogen products is as high as 70 % or more. Thus, the quality of the hydrogenation product as a plasticizer may be improved. And, a reaction may uniformly occur inside of the reactor, and catalysts at the upper/lower part of the reactor may be uniformly loaded, thus significantly improving catalyst life.

After the reaction is completed, a liquid phase hydrogenation product and a non-reacted gas phase raw material are separated. The separated gas phase raw material may be recycled to the hydrogenation process, while the recovered hydrogenation product may be subjected to pressure reduction and cooling processes and finally separated.

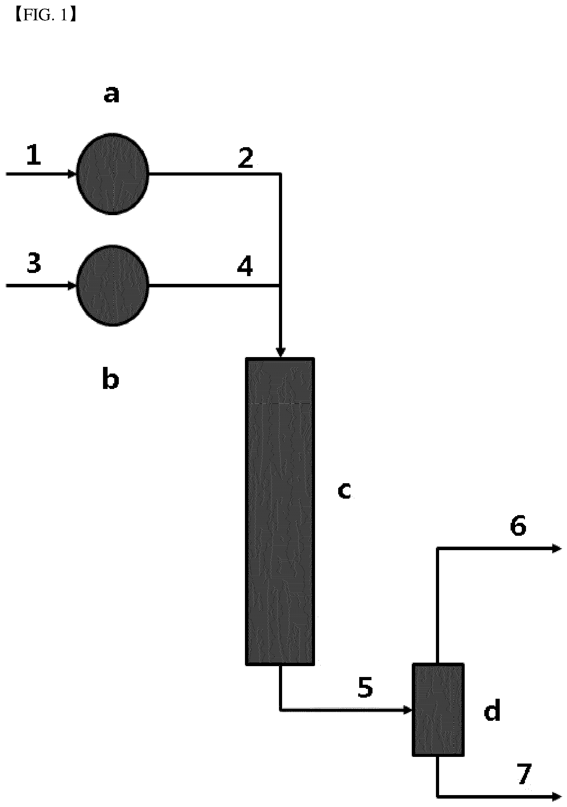

FIG. 1 is a drawing illustrating the hydrogenation apparatus used in the hydrogenation method of the invention.

Referring to FIG.1, the hydrogenation apparatus may consist of heat exchangers (a, b), a reactor (c), a gas phase-liquid phase separator (d), and the like.

The heat exchangers (a, b) function for raising a temperature before introducing a gas phase raw material (1) and a liquid phase raw material (3) into the reactor(c), and may be omitted according to necessity.

The gas phase raw material (2) and the liquid phase raw material (4) are introduced into a tubular-type reactor (c) filled with a hydrogenation catalyst, thus progressing hydrogenation. The reactor may further include an external jacket for removing heat, so as to remove reaction heat. Herein, the gas phase raw material (2) may be fed from the upper part or lower part of the reactor, and the liquid phase raw material (4) may be fed from the upper part of the reactor.

Since hydrogenation of a phthalate compound causes a reaction between the gas phase raw material and the liquid phase raw material on the solid catalyst surface, the liquid phase raw material should be flowed at a flux capable of forming a film on the solid catalyst surface. Thus, it is appropriate to gradually flow the liquid phase raw material from the upper part by gravity. On the contrary, in case the liquid phase is introduced from the lower part, the solid catalyst may be submerged by the liquid reactant, and thus it may become difficult for the gas phase reactant to penetrate the solid catalyst, which is not appropriate.

The reaction mixture (5) discharged from the reactor (c) is transferred to a gas phase-liquid phase separator (d), in which the liquid phase reaction product (7) and the gas phase non-reacted material (6) are separated. The separated reaction product (7) may be recovered and subjected to an additional purification process, and the gas phase non-reacted material (6) may be recycled for discharge or reuse.

However, in FIG. 1, the position of each piece of equipment may be changed, and if necessary, other equipment not shown in FIG. 1 may be included, and thus the hydrogenation method of the invention is not limited to the equipment and process sequence shown in FIG. 1.

According to the hydrogenation method of the invention, stereoselectivity of hydrogenation may be improved to obtain a reaction product having a cis isomer content of 70 % or more. The product exhibits excellent plasticization efficiency and rapid absorption speed, and excellent product transparency after solidification, and generates less leaching on the product surface even after use for a long time, thus exhibiting excellent properties as a plasticizer.

The hydrogenated phthalate or terephthalate compound thus prepared may be usefully used as a plasticizer. Specifically, a plasticizer including the phthalate or terephthalate compound may be used for a stabilizer, paint, ink, a liquid blowing agent (masterbatch type), an adhesive, and the like.

The phthalate or terephthalate compound prepared according to the above method has excellent purity and a high cis isomer content, and thus has excellent quality as a plasticizer. Therefore, it may be appropriately used for a plasticizer of a resin selected from ethylene vinyl acetate, polyethylene, polypropylene, polyvinyl chloride, polystyrene, polyurethane, polybutadiene, silicon, a thermoplastic elastomer, or a copolymer thereof.

A resin composition including the phthalate or terephthalate compound prepared according to the above method as a plasticizer, and including the above resin, may be used for various products. For example, it may be used for the preparation of a film for food packaging (for example, a wrap), an industrial film, a compound, a decosheet, a decotile, a soft sheet, a hard sheet, a wire, and a cable, wall paper, a foaming mat, artificial leather, flooring, a tarpaulin, gloves, a sealant, a gasket for a refrigerator, a hose, a medical instrument, a geogrid, a mesh tarpaulin, toys, stationery, an insulating tape, a coating for clothes, PVC labels used for clothes or stationery, a stopper liner, stoppers for industrial or other uses, an artificial bait, parts in electronic equipment (for example, a sleeve), an automobile interior material, an adhesive, a coating, and the like, but is not limited thereto.

Hereinafter, the actions and effects of the invention will be explained in more detail through specific examples. However, these examples are no more than illustrations, and the scope of the right of the invention is not determined thereby.

### <Example>

### Example 1

Dioctyl terephthalate (DOTP) having purity of 99 % was introduced into a reactor, and hydrogenation was conducted at a reaction pressure of 150 bar and a reaction temperature of 165 to 175 °C.

The mass flux of DOTP per unit area of the reactor (m²) was 20,000 kg/h, and hydrogen was introduced at a hydrogen/DOTP mole ratio of 6.

The reactor was in the form of a single-lumen tube, the total length of a part filled with a catalyst in the tube was 3.0 m, and a cooling fluid (Therminol 55) was flowed to the external jacket of the reactor to control such that a temperature deviation of the reactor was maintained at 15 to 25 °C during the reaction (a temperature deviation per unit length at 5 °C/m). In addition, the reaction amount per unit volume (m³) of the reactor during the reaction was set up as 30 kmol/h or less.

The catalyst used in the reactor was a ruthenium (Ru) catalyst having a ruthenium content of 0.5 wt%, based on 100 wt% of an alumina (Al₂O₃) carrier, and a cylinder type of catalyst having a diameter of 3 mm and a height of 3 mm was used.

### Example 2

Hydrogenation was conducted by the same method as Example 1, except that the reaction temperature was maintained at 155 to 165 °C, and the temperature deviation of the reactor was maintained at 7 to 16 °C during the reaction.

### Example 3

Hydrogenation was conducted by the same method as Example 1, except that the reaction temperature was maintained at 140 to 155 °C, and the temperature deviation of the reactor was maintained at 8 to 17 °C during the reaction.

### Comparative Example 1

Hydrogenation was conducted by the same method as Example 1, except that the temperature deviation of the reactor was maintained at 25 to 35 °C during the reaction.

### Comparative Example 2

Hydrogenation was conducted by the same method as Example 1, except that the temperature deviation of the reactor was maintained at 35 to 40 °C during the reaction.

### <Experimental Example>

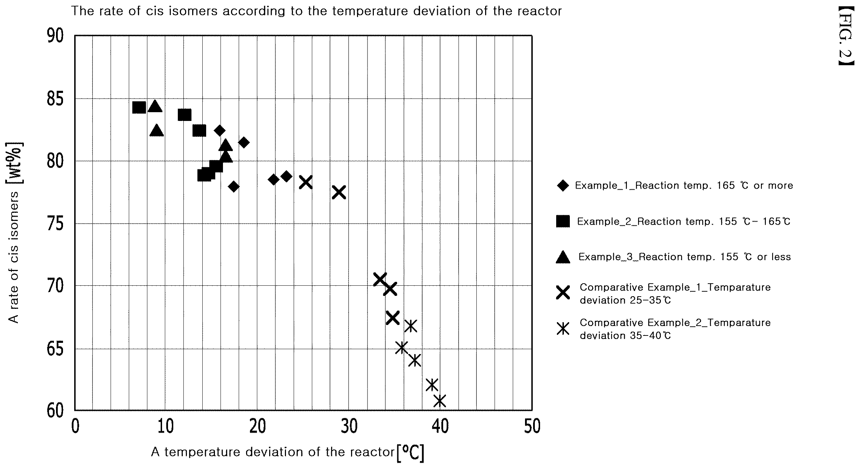

After completing the hydrogenation reactions of Examples 1 to 3 and Comparative Examples 1 and 2, an unreacted gas phase raw material was separated from the reaction mixture to obtain a hydrogenation product. For the examples and comparative examples, the contents of cis isomers in the hydrogenation products were measured, and the results are shown in FIG. 2. The content of cis isomers was quantified using a gas chromatography device. Specifically, when standing at 40 °C for 4 minutes, and then heating at 70 °C to 280 °C for 24 minutes, the content of cis isomers was reported as 25.5 minute, and the content of trans isomers was reported at 26.0 minute.

Referring to FIG. 2, it can be seen that in case a temperature deviation of the reactor is 30 °C or less as in Examples 1 to 3, irrespective of a reaction temperature difference, the contents of cis isomers in all the products was 75 % or more.

However, in the case of Comparative Examples 1 and 2 wherein a temperature deviation of the reactor exceeds 30 °C, stereoselectivity of the reaction remarkably decreases, and the rate of cis isomers decreases. Comparing Comparative Examples 1 and 2, it is confirmed that as the temperature deviation increases, the content of cis isomers continuously decreases.

From the experimental results, it is confirmed that by controlling a temperature deviation in a reactor during the hydrogenation of a phthalate compound, stereoselectivity of the reaction may be significantly improved, and it can be seen that according to the method of the invention, the content of cis isomers is high, thus obtaining a hydrogenation product having excellent quality as a plasticizer.

### [Reference numerals]

a, b: heat exchangers
c: reactor
d: gas phase-liquid phase separator
1, 2: gas phase raw material
3, 4: liquid phase raw material
5: reaction mixture
6: gas phase un-reacted material
7: liquid phase reaction product

## Claims

1. A method for the hydrogenation of a phthalate compound comprising the step of reacting a phthalate compound with hydrogen in the presence of a hydrogenation catalyst in a reactor,
wherein a temperature deviation in the reactor is 0 to 30 °C during the reaction, and
the content of cis isomers in the hydrogenation product separated after the reaction is 70 % or more.

2. The method for the hydrogenation of a phthalate compound according to claim 1, wherein during the reaction, a temperature deviation per unit length (m) of the reactor is 10 °C or less.

3. The method for the hydrogenation of a phthalate compound according to claim 1, wherein the reaction amount per unit volume (m³) of the reactor during the reaction is 100 kmol/h or less.

4. The method for the hydrogenation of a phthalate compound according to claim 1, wherein the reactor includes a cooling member in which coolant is circulated, installed outside.

5. The method for the hydrogenation of a phthalate compound according to claim 1, wherein the mass flux per unit area (m²) of the phthalate compound introduced into the reactor is 10,000 to 100,000 kg^{∗}h^{-1∗}m⁻².

6. The method for the hydrogenation of a phthalate compound according to claim 1, wherein the amount of hydrogen introduced into the reactor is 3 to 300 mol, per 1 mol of the phthalate compound.

7. The method for the hydrogenation of a phthalate compound according to claim 1, wherein the phthalate compound is one or more selected from the group consisting of phthalate, terephthalate, isophthalate, and carboxylic acid compounds thereof.

8. The method for the hydrogenation of a phthalate compound according to claim 1, wherein a gas phase raw material is fed from the upper part or lower part of the reactor, and a liquid raw material is fed from the upper part of the reactor.

9. The method for the hydrogenation of a phthalate compound according to claim 1, wherein the hydrogenation catalyst is one or more selected from the group consisting of ruthenium (Ru), rhodium (Rh), palladium (Pd), and platinum (Pt).

10. The method for the hydrogenation of a phthalate compound according to claim 1, wherein the amount of the hydrogenation catalyst is 3 wt% or less, based on 100 wt% of a carrier.

11. A hydrogenated phthalate or terephthalate compound, prepared by the method according to any one of claims 1 to 10.

12. A plasticizer comprising the hydrogenated phthalate or terephthalate compound of claim 11.

13. A resin composition comprising the plasticizer of claim 12, and a resin selected from ethylene vinyl acetate, polyethylene, polypropylene, polyvinyl chloride, polystyrene, polyurethane, polybutadiene, silicon, a thermoplastic elastomer, or a copolymer thereof.
